# EUROPEAN PATENT APPLICATION

(11) **EP 3 415 123 A1**
(43) Date of publication of application: **19.12.2018**
(21) Application number: 16877032.9
(22) Date of filing: 20.06.2016
(51) Int. Cl.: A61F 4/00, A61H 1/00, A63B 23/00, A63B 24/00

(54) **SYSTEM AND METHOD FOR RESTORING HUMAN MOTOR ACTIVITY**

(30) Priority: 23.12.2015 EA 2016001
(71) Applicant: Limited Liability Company Kinideks, Moscow Region, 141407 (RU)
(72) Inventor: TSAROU, Ivan Mikhailavich, Minskiy District. Minsiy Reg. 223060 (BY); LUKASHEVICH, Uladzislau Anatolievich, Minsk, 220136 (BY)
(74) Representative: Smirnov, Alexander
(86) International application number: PCT/BY2016/000004
(87) International publication number: WO 2017/106953

(57) **Abstract**

The claimed invention relates to a system for human motor function recovery created based on a robotic kinetic trainer containing a whole-body controlled exoskeleton, as well as to a method of human motor function recovery based on adaptive kinesitherapy techniques. A system for human motor function recovery is offered, implemented as a robotic kinetic trainer with a whole-body exoskeleton, formed by a controlled movable frame, consisting of kinematically interconnected orthopaedic modules with a suspension system that correspond to body parts, actuated by the drives subsystem, and an outer rigid fixed frame, kinematically connected to the controlled movable frame and forming a two-layer exoskeleton together with it. Electromechanical drives subsystem, where each of the drives is connected to the corresponding orthopaedic module by flexible communication, is located on the outer fixed frame. Orthopaedic modules and human body physiological indicators sensors are connected to the control software and hardware for spatial position change of each orthopaedic module via the outer fixed frame. The hardware and software are also equipped with a virtual reality imaging subsystem configured to co-operate with the electromechanical drives subsystem. The two-layer exoskeleton is equipped with the initial suspension position fixation device with support in the hip area and/or in the upper body area. A method of human motor function recovery is also offered, where the recovery of the system described above is carried out in two stages, where at the first stage, stable motion pattern matrices are created or restored in the central nervous system, and at the second stage, the connections between the surrounding events and the recovered motion pattern matrices are restored as responses to these events.

## Description

### Field of the Invention

A claimed invention relates to medicine, in particular, to the system of recovering human motor activity designed on the basis of a robotic kinesiological training device containing a complete controllable human exoskeleton. The invention also relates to the method for recovery of human motor activity based on adaptive kinesitherapy techniques. The claimed device and method can be used to recover motor activity in cases of diseases accompanied by motor disorders, in such fields as rehabilitation (restorative medicine); traumatology and orthopedics; neurology (pediatric neurology), neurorehabilitation (postoperative rehabilitation); geriatrics; sports medicine.

### Prior Art

An idea of an exoskeleton, a device that enables people to mechanically enhance the strength of their limbs, has already been developing for several decades. This idea was initially proposed by the military, primarily for increasing the stamina and combat efficiency of soldiers and providing them with super powers. The fact that civilian experts joined the development of this idea made it possible to significantly expand the possible application areas for exoskeletons-from the creation of special spacesuits for work under overload conditions to their use as devices for the rehabilitation of patients, in particular, for the recovery of walking function in people with injuries and various diseases of the central nervous system in general. Exoskeletons used for medical (rehabilitation) purposes are currently being actively developed by the experts from Israel, Switzerland, Germany, the USA, Russia and some other countries. They mainly develop exoskeletons for separate limbs, in particular, hands [1], and/or extremity girdles-the girdles of the lower or upper extremities. Exoskeletons of separate extremities have a very narrow specific sphere of application: they are most often used as robotic manipulators, however, in principle, we cannot exclude their use for the recovery of the motor activity of a limb(s) lost as a result of an injury or a neurological disease.

In the same way, the exoskeletons of the extremity girdles have a limited functionality and can be used for the recovery of the motor activity only of the corresponding girdle of upper or lower extremities. Thus, we know the ReWalk (meaning "to walk again") exoskeleton produced by the Israeli ARGO company: the device represents a suit worn by the patient on his/her lower limbs and on the waist [2]. The exoskeleton itself is a system that is integrated into one single entity and driven by miniature electric motors, it is also equipped with the sensors that enable the device to determine what the patient wants at this particular moment: to walk, to sit, to climb the stairs, etc. The device is powered by a special battery borne on the patient's back in a special backpack. One battery charge can ensure continuous operation of the exoskeleton for almost four hours. The patient can choose the appropriate mode of operation for the exoskeleton himself/herself, however, in order to make the device start moving, the patient must lean forward and change the position of the crutches. Today there are several development projects of the ReWalk exoskeleton, some of them make it possible to adjust the device in accordance with the patient's height and take into account the individual features of the patient's body. However, this device cannot be used in cases of severe disturbances of motor activity, primarily related to neurological diseases caused by brain damage, since the device has no such function to maintain the patient's body in an upright position and in equilibrium. Besides, the targeted treatment of motor activity of the whole organism is not provided, despite the fact that virtually the entire human musculoskeletal system is involved in the process of performing a "simple" step.

In consideration of the above note, the most part of exoskeletons of the girdle of lower extremities known by us are used mainly only in traumatology and orthopedics.

More complicated exoskeletons are also known, for example, the EXOATLET [3] exoskeleton intended for vertical orientation and walking of a patient with locomotor disorders of the lower extremities, this exoskeleton is suitable for patients with a very wide range of diseases. This exoskeleton has a control system based on the signals of the force/torque sensors and electromyogram. Control algorithms enable the patient to move in an automated mode with the replication of the most natural pattern of human walking, which makes it possible to significantly accelerate the process of recovery of motor and nervous activity.
Despite the expansion of possibilities of the use of this exoskeleton, in particular, of the possibility of its use for the recovery of motor activity of people with various neurological diseases, the targeted treatment, just like in the cases described above, affects only the girdle of lower extremities. In addition, the feedback used in the exoskeleton under consideration, does not provide sufficient "flexibility" for the exoskeleton to adjust various characteristics of the patient's movement (elevation angle of the leg, the speed of steps performance, the ability to abduct the leg to the sides). In fact, this exoskeleton enables the patient to perform only the simplest movements.

The "partial" exoskeletons described above can preferably be used individually outside of special treatment or rehabilitation facilities and without constant monitoring on the part of specialists, since, first of all, they provide people with mobility and "maneuverability"-the possibility of "independent" movement without the use of wheelchairs and other auxiliary aids. Taking into account the peculiarities of the use of such exoskeletons, one of the requirements imposed on them is a relatively small weight, which significantly reduces the possibilities for expansion of the exoskeleton's functionality. The lack of constant monitoring by specialists, as well as a rather limited "feedback" function provided by such devices do not make it possible to make amendments to the recovery program, which significantly reduces the recovery efficiency.

"Full-body" exoskeletons seem to be more promising, from the point of view of efficiency of the recovery of the motor activity and expanding the area of controllable impact with coverage of virtually whole human organism.

Thus, it is known that the automated device by the Swiss Hocoma AG company designed according to the principles of the exoskeleton, is used for the therapy of "paretic" or "semi-paretic" patients, in combination with the treadmill. The device automatically moves the patient's legs on the treadmill and consists of power driven and controlled bioprostheses that control the legs in accordance with the physiology of movements, of a treadmill and a safety device [4]. Knee and femoral junctions of bioprostheses contain drives. The bioprosthesis is stabilized in relation to the treadmill with the use of stabilization means in such a way that the patient does not have to maintain equilibrium himself/herself.

It also known from the prior art, the LokomatPro advanced robotic complex for locomotor therapy with extended feedback produced by the above-mentioned Swiss Hocoma AG company [5]. The mechanical part of the complex is a full-body human exoskeleton formed by a controlled movable frame consisting of kinematically interconnected orthopedic modules of body parts that can be attached to the corresponding parts of the body and powered by a subsystem of drives. The complex also contains firmware for the control over the changes of position of each orthopedic module in space, executed on the basis of a computer with a controller with the possibility of controlled communication with each orthopedic module through the corresponding drive. LokomatPro combines functional locomotor therapy with motivation and assessment of the patient's condition through advanced feedback tools and virtual reality. An integrated feedback system monitors the patient's gait and is visually displayed in a real time mode, increasing the patient's motivation and encouraging him/her to actively participate in the process. To recover the motor activity of a person, an individual training motor recovery program is composed with the use of LokomatPro with allowance for each patient's diagnosis, his/her current physiological state, and motor stereotypes are formed in the patient's central nervous system according to a given program by the means of forced changes of the positions of corresponding body parts in space with the help of the LokomatPro complex that includes an exoskeleton associated with the computer-based control means.

In a way similar to the described LokomatPro system, the ReoAmbulator robotic complex by the Israeli Motorika company [6] has been created and is used to recover human motor activity. The complex uses an exoskeleton with a unique design of extensible robotic ortheses, which allows the doctor to easily adjust them to the desired length of legs and width of the patient's pelvis. At the same time, firmware calculates the appropriate step length, depending on the patient's height, which makes it possible to apply ReoAmbulator both to children beginning from 2.5 years (90 cm) and adults (up to 202 cm). A special program titled "Walking analysis" offers the patient a step pattern (footprints on the path), and the doctor teaches the patient to control his/her steps, contributing to the development of the correct stereotype of movements. In addition, the complex can work in various training modes. Robotic (passive) mode is used for the patients who are unable to actively move and forms a correct walking stereotype. In the initiated (active-passive) mode the patient begins to move, and the robot helps him/her finish any movement, even if the patient is not functionally ready for this at the moment, continuing to form the correct stereotype of walking. The next, accelerating (active-passive) mode motivates the patient to make constant efforts, forcing the robotic ortheses to move, and the robotic mode is turned on, if necessary. And, finally, the active (independent) mode involves the possibility not only to move forward, but also to elaborate lateral movements, moving backwards, which helps to train the patient's coordination and simulate the situations that can occur in everyday life.

Just like in case of the LokomatPro complex, the method for recovering the human motor activity implemented through it includes the composition of an individual training motor recovery program and the formation of motor stereotypes in the central nervous system by a prescribed program by forced changes in the positions of corresponding body parts in space with the use of the ReoAmbulator complex.

One of the main disadvantages of the two complexes described above, despite their high level of automation and adaptation to each patient, is the insufficient number of degrees of freedom provided by individual orthopedic modules and exoskeleton in general. This is explained by the peculiarities of the design of orthopedic modules and their significant cumbersomeness. In addition, in the complexes considered the patient's vertical orientation is achieved by the system of "suspension" of the patient's body, which ensures the stability of the body position in its vertical state only due to the body weight. However, upon performance of movements that simulate walking and in case of inaccurate calculation of the body weight unloading value, a destabilizing effect of the body position emerges, in particular, in the areas corresponding to separate parts of the spine, which leads to the formation of an erroneous movement stereotypes (without taking into account the coordination of all parts of the body during the movement performance). In addition, the channels available for obtaining information by the patient (in particular, the visual channel, etc.) are involved in the process of formation of motor stereotypes and the complex feedback at an insufficient degree. With allowance for this, first of all, taking into account the "weak" feedback during the implementation of the recovery method with the use of the complexes under consideration, the problem of effective formation/recovery of stereotypes of complicated movements in patients remains unresolved. Besides, the design of individual orthopedic modules and exoskeleton in general do not provide the possibility of development of the movements of separate joints.

### Summary of the Invention

An analysis of the level of technology has showed that the system of ReoAmbulator [6] described above and the method implemented with its use are the closest to the claimed system in the combination of general technical specification and in the method for recovery of human motor activity. The specified complex (system) and method can be adopted as prototypes for the system claimed within the frame of this application and for the method of recovery of human motor activity.

Thus, the objective of the invention is to provide a system for the recovery of human motor activity in the form of a robotic kinesiological training device containing a full-body exoskeleton of a person, as well as the development of a method for the recovery of human motor activity with the use of such a system. The system and method should have a higher efficiency due to an increase in the intensity of recovery of motor activity, both in separate joints and the whole human musculoskeletal system with regard to both simple and complex movements. The system and method should also provide the possibility of rehabilitation at the earliest stages, wherein controlled dosed "movement development" of joints and the formation of motor stereotypes are performed in bedridden patients, i.e. in the prone body position.

The assigned task is solved by the claimed system of recovery of human motor activity in the form of a robotic kinesiological training device containing a full-body human exoskeleton including a controlled movable skeleton consisting of kinematically interconnected orthopedic modules of corresponding body parts that can be attached to the corresponding body parts and put into motion by a subsystem of drives, firmware means for the control over the changes of the position of each prosthetic module in space, designed on the basis on a computer with a controller with a possibility of controlled communication with each orthopedic module through the corresponding drive, as well as feedback means based on at least one sensor of at least one physiological parameter of the human body. The assigned task is solved due to the fact that the controlled movable frame is kinematically connected with the external rigid stationary frame made in the form of a volumetric frame structure determining the space for the changes in the position of orthopedic modules and forming a two-layer exoskeleton with a controlled movable frame. A subsystem of electromechanical drives is placed on the external stationary frame, each of the drives is connected to the corresponding orthopedic module by the means of flexible connection. Each orthopedic module and each sensor of physiological indicators of the human body is connected with the firmware means of control over the changes in the position of each orthopedic module in space through an external stationary frame. The firmware means of control over the changes in the position of each orthopedic module in space contain an additional virtual reality visualization subsystem that, jointly with the subsystem of electromechanical drives, gives a possibility of coordinated functioning. The external stationary frame is provided with means of fixing the initial position of the body in a suspended state with the support in the hip region and/or in the upper body.

As the matter of fact, the claimed system of recovery of human motor activity made in the form of a robotic kinesiological training device is a training simulator of complex human movements-a firmware complex performing individually dosed (by two functional sections: "4-stage dosing by physical factors" and "5-stage neurobiomechanical dosing") and dynamically controlled simulation of basic human movements. The implementation of this function is ensured by the launch of motor "augmenting programs" representing various motor patterns in the form of "Assist" models. High rehabilitation efficiency with regard to the recovery of motor activity is achieved by combining the software and mechanical components with the virtual reality visualization system into a single rehabilitation complex.

In this case, the mechanical component includes the following elements:
- whole-body exoskeleton, formed by a controlled movable frame, consisting of kinematically interconnected orthopaedic modules with a suspension system that correspond to body parts;
- outer rigid fixed frame, made as a volumetric frame structure (gravity frame);
- electromechanical drives subsystem.

Two-layer exoskeleton design, formed by a movable frame, kinematically connected to the outer rigid fixed frame, allows for better exoskeleton customization to different requirements, significantly expanding its scope of application (diseases and conditions where rehabilitation is possible).

Hardware and software, which include the virtual reality imaging system, provide for:
- specific virtual reality environment;
- visualizer;
- personal computer;
- controller.

In terms of functionality, the claimed human motor function recovery system, designed as a robotic kinetic trainer, combines a number of functional units that carry out independent directions in modern kinesitherapy system, namely:
- android mechanisms-exoskeleton;
- hardware and software for stepping locomotion recovery;
- hardware for individual joints mechanotherapy;
- hardware and software for muscle tone recovery exercises;
- massage devices.

Presence of flexible connections of the controlled movable frame to the outer rigid fixed frame (supporting gravity frame), that has own electromechanical drives (gravity dosing system) allows for the following functions:
- facilitating patient lifting;
- gravity load/discharge dosing;
- ensuring a certain patient's spatial position and selected directions for the necessary movement;
- environmental augmentation with displacement over 6 degrees of freedom (roll, pitch and yaw);
- potential connection to a full-fledged virtual reality system.

In terms of the invention, augmentation means a combination of factors contributing to deepening the concept of the action performed. An artificially transformed environmental factor that adapts motor task performance to the individual motion space act as an augmentator. Fundamentally, environmental augmentation is designed to stabilize the perception system, by lowering the jumps in the quality parameters of environment fluctuations.

The main task that the claimed human motor function recovery system allows to deal with, involves proportionate start of complex training motions from simple, performed in lying and standing positions, to more complex ones, which can be performed with and without external support. Fixtures and cushioning elements allow for safe practice with regard to neuromuscular and osteoarticular systems. The main goal of such practices is to restore compound motion image in the brain that can be performed by the body, i.e. the motion pattern. In turn, the main goal of the motion pattern is to create walking and posture automatism, a synergistic distribution of different-purpose muscle activity in maintaining the posture and motor function.

When restoring motor function, it should be borne in mind that no motion in a large joint within a month leads to nearly complete erasure of the idea of this motion in the brain, that results in no inflow from the brain to the joint. The claimed system allows solving an important task related to the development of articular contractures (joint stiffness) at the earliest stages of recovery, using the Constant Passive Motion therapy, while in addition to mild load dosing, the kinetic trainer (in various embodiments) performs massage and vibration activation of the muscles, that have lost the ability to contract (vibration practice). Coordination practice is the next task that can be solved with the kinetic trainer. Coordination function is fundamental when performing a complex action. A simple movement, for example a knee-jerk reflex or hand pulling away from fire, can be a result of a motor command carried from the center to the periphery. However complex motor actions, designed to solve a problem or achieve some result, do not occur this way. Experts know that the result of any complex motion depends not only on the actual control signals, but also on a range of additional factors that introduce deviations into the planned course of motion and cannot be predetermined. As a result, the final motion goal can only be achieved if it is constantly adjusted or corrected. And for that to happen, the central nervous system (CNS) should know the actual state of the current motion. In other words, the afferent signals with information about the real course of motion should be continuously sent to the CNS, and then processed into correction signals. Within the claimed system and the claimed method, which will be discussed in detail below, special and theoretically optimal moving elements activation sequences in unpredictable variants lead to activation of cerebellum pathways receptors (and related brain structures responsible for coordination).

An action of the abovementioned additional factors dictates the need for continuous taking account of information on the locomotor system state and actual course of motion. This information is called "feedback signals." N.A. Bernstein described the role of feedback signals in motor control, as in control tasks in general, long before similar ideas emerged in cybernetics. The thesis that motion cannot be performed not taking into account the information about it is factually based. At the same time, it is important that not only the professional performing rehabilitation receives "feedback signals" during the movement, but also the patient himself, as was mentioned above. The visual channel is one of the most effective to send the "feedback signals" to the patient. For these purposes, control hardware and software for spatial position change of each orthopaedic module of the claimed system are also equipped with a virtual reality imaging subsystem configured to co-operate with the electromechanical drives subsystem and display motion for the patient.

### Preferred embodiments

In preferred embodiments of the claimed system, the virtual reality imaging subsystem has at least one visualizer, wherein the control hardware and software for spatial position change of each orthopaedic module are also equipped with a unit for creating at least one specific virtual reality environment connected to the visualizer to generate motor images.

The term "specific virtual reality environment" used in the present description means a block of software and hardware providing the creation of correct motion illusion (spatial image) in the brain, i.e. creation of "motion pattern" based on "motor motion image". In this case, the brain perceives this illusion as a full-fledged motion.

In other preferred embodiments of the claimed human motor function recovery system, each orthopaedic module can be made with an option for mechanotherapy of individual joints. The concept of "individual joints mechanotherapy" is based on the classical meaning of "mechanotherapy," but at the same time "mechanotherapy" can be carried out in joint-specific mode (load intensity, angular motion range, etc.), set by control hardware and software for spatial position change of relevant orthopaedic module, particularly based on analysis and processing of data received from feedback means. Moreover, orthopaedic modules can be designed to transmit vibrational and/or massaging influences to respective body parts. This, as already mentioned above, allows for vibration practice starting from the earliest stages of recovery, thereby solving an important task related to joint contractures development by the CPM therapy.

Preferred embodiments of the claimed system also include those in which the controlled movable frame is equipped with at least one additional element selected from the group consisting of at least the fixtures and cushioning elements, as well as customization elements. For instance, controlled movable frame can have pelvic, thoracic and other fixatives, as well as a cushioning pad mounted on the support.

In preferred embodiments of the claimed system, the kinematic connection between the orthopaedic modules can be ensured by electromechanical parts. Generally, electromechanical parts can be effected based on electric motors connected with the levers installed between them. In this case, each electromechanical part corresponds to a certain module: femoral, knee, etc., and besides the electrical motor can have various sensors (rate-of-turn, motor temperature, etc.) connected to the motor or the drive shaft, as well as the gear installed on the drive shaft. Depending on the specific module, there can be an articulated joint between the levers.

As already mentioned above, body support in the hip area as part of the initial suspension position fixation device is essential for the claimed system. In this case, preferably, there is lower body support in the perinea region. This embodiment of the initial body position fixation device, taking into account the aforementioned preferred presence of a cushioning element-a cushioning pad-mounted on the support, not only provides a more comfortable patient's condition, but also an absolutely stable vertical position of the patient's body during movement, secured without additional patient's effort, which is especially important at the early stages of rehabilitation.

In alternative preferred embodiments of the claimed system, the initial suspension position fixation device can be designed with a possible upper body support, for example, in the axillary region. As in the embodiment described above, it is also preferable to have cushioning elements--cushioning pads mounted on axillary supports.

In other alternative preferred embodiments of the claimed system, the initial suspension position fixation device can be designed separately with a possible upper body support and lower body support in the perinea region with the possibility to adjust the distribution of the fixation percentage between the upper and lower parts. Such fixation provides a high degree of optimal patient's gravity discharge (especially taking into account the possibility of automatic or automated calculation of the fixation percentage distribution), compensating for the spinal load caused by body weight, which is crucial for the rehabilitation of patients with spinal injuries, etc.

Preferred embodiments of the claimed system for motor function recovery also include those where the body fixation devices are equipped with cushioning components facilitating passive adduction of the body part towards at least one point of the rigid fixed frame. Fixation devices with cushioning components in conjunction with flexible, and possibly elastic, connections allow the patient to comfortably "control" his motions according to the "puppet" principle, ensuring a sufficient number of degrees of freedom, which increases the sense that motion performed under exoskeleton's orthopaedic modules "control", is real and natural.

Preferred embodiments of the claimed system for motor function recovery also include those where the control hardware and software for spatial position change of each orthopaedic module are designed to remotely record and/or correct the program for the coordinated functioning of the virtual reality imaging subsystem and the electromechanical drives subsystem. Generally, exoskeleton can be programmed remotely (via the Internet), or directly in the presence of the patient at kinetic trainer's location. Remote programming (Remote Trainer mode) provides for obtaining full information remotely, both about the performance process of the tasks set, and about the assessment of practice quality parameters, based on what it is possible to make timely adjustments to kinetic trainer's settings. When programming directly at kinetic trainer's location (Virtual Trainer mode), kinetic trainer's training program is adjusted automatically: the program independently changes the dosage and practice mode. There can also be an Individual Dosing function, which will allow the patient to adjust practice loads independently, based on expert recommendations, which is highly important for the claimed system embodiments for individual use at home.

In a number of preferred embodiments of the claimed system for human motor function recovery, particularly for the purpose of the abovementioned Individual Dosing function, the control hardware and software for spatial position change of each orthopaedic module may also have a control module in the form of a mechanic arm, designed with a possibility of manual control by the trainee by a spatial position change of each orthopaedic module.

The stated task is also achieved by the claimed method for human motor function recovery, including preparation of an individual training motor recovery program and creation of motion pattern in the central nervous system according to the prepared program by forced relevant change of the spatial position of body parts using the human motor function recovery system, including the exoskeleton connected to computer-based controls. The task is solved by using the above claimed system as a system for human motor function recovery. That said, recovery is carried out in two stages. At the first stage, stable motion pattern matrices are created or restored in the CNS. At the same time, a motor image of at least one virtual motion is generated (in accordance with the individual training motor program), which is visualized and transmitted through the visual channel to the trainee's CNS, while a control action corresponding to this motion is transmitted to the controlled movable frame of the exoskeleton that forces at least one corresponding body part to move over and over again. At the second stage, the connections between the surrounding events and the recovered motion pattern matrices are restored as responses to these events. At the same time, a motor image of at least one virtual event requiring a motor response is generated, which is visualized and transmitted through the visual channel to the trainee's central nervous system, followed by a control action corresponding to this motor response transmitted to the controlled movable frame of the exoskeleton that forces at least one corresponding body part to move over and over again. By doing so, the trainee's condition, in particular the condition of the locomotor system, is monitored using feedback.

The claimed method of human motor function recovery is the basis for a new scientific direction, designated as "adaptive kinesitherapy," aimed at increasing the social adaptation of persons with reduced mobility through neurosensory reprogramming in their spatial orientation control system. Adaptive kinesitherapy means a motor disorder occupational therapy method, based on creating augmented "rigid matrices" of motion patterns intended for effective spatial orientation (external space use). When performing training motions, time-matched information received by brain from the complex receptor device integrates with the information from virtual reality environment, leading to illusory representation (illusion) of the real motion in virtual reality environment. At the same time, the training/rehabilitation process organized in accordance with the claimed method ensures that a patient creates a number of basic motion patterns as "rigid matrices," the arbitrary combination of which allows changing the structure of the patient's motor skill: as programming a new skill or correcting it.

In various preferred embodiments of the claimed method of motor function recovery, the training motor program is prepared taking into account the load dosage and complicating the training motions starting with simple ones, performed in lying or standing position, and/or in a way that motor images are created by complicating them from static and statnamic to dynamic ones. This ensures the possibility of continuous patient rehabilitation from the very early stages to the completion of the course.

Training load is preferably dosed based on previous and current practice results, as well as on dynamic (determined during the practice) assessment of the trainee's locomotor system active structures viscoelasticity in automatic or manual mode. Basically, such traditional approach to load dosing within the claimed method allows optimizing the training motor program for each patient individually according to his or her current physical condition. At the later stages of rehabilitation in the claimed method of motor function recovery, for various options of creating the motion patterns for more complex motions associated with a number of special restrictions, the training motor program is preferably set:
- taking into account motion space limitations due to the virtual component, which directs the virtual motion towards the motion space limitation, thus forming an idea in the patient's CNS of the potential readiness of the locomotor system to use this part of the motion space;
- taking into account motion space limitations due to the controlled movable frame of the exoskeleton, using which the practiced motion is oriented towards the motion space limitation, thus forming an idea in the patient's CNS of the potential ability of the locomotor system to use this part of the motion space;
- taking into account motion space limitations due to the virtual component and the controlled movable frame of the exoskeleton, which together strengthen the motion matrix oriented towards motion space limitation.

Preferred embodiments of the claimed method of motor function recovery also include those providing for the active practice with overcoming the resistance of the cushioning components facilitating passive adduction of a body part due to an arbitrary movement of the body part in at least one direction of the motion pattern matrix.

### Short Description of Drawings

The above and other advantages and benefits of the claimed system and method of human motor function recovery will be further discussed with reference to the figure positions in the drawings, where some possible preferred, but not limiting embodiments of the claimed system are presented as a whole and individual system components, as well as some non-limiting examples of the recovery method.

In particular, the drawings outline:
Figure 1 - general view of one of the embodiments of the claimed system (with a patient);
Figure 2 - rear view of the exoskeleton (controlled movable frame) in one of the embodiments of the claimed system (with the patient);
Figure 3 - side view of the exoskeleton (with the patient);
Figure 4 - general view of the rigid fixed frame in one of the embodiments (with the patient);
Figure 5 - side view of Figure 4 frame;
Figure 6 - bottom view of Figure 4 frame;
Figure 7 - scheme of the claimed method's "motor programming" principle;
Figure 8 - scheme of the claimed method's "motor control" principle;
Figure 9 - simplified hardware and software flowchart;
Figure 10 - the algorithm for determining the viscoelasticity of the locomotor system articulations.
Figure 11 - the algorithm for automatic selection of exercises performed on two legs (two-point exercises) and one leg (one-point exercises).

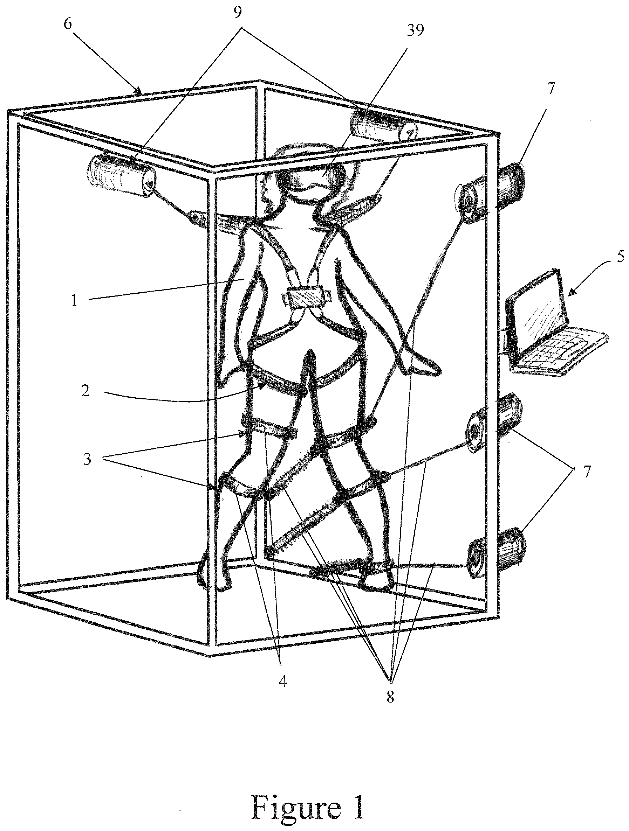

Figure 1 shows a general view of one of the embodiments of the claimed system for human motor function recovery with a patient. The system is designed as a robotic kinetic trainer containing a whole-body exoskeleton 1. The exoskeleton is formed by a controlled movable frame 2, consisting of kinematically interconnected orthopaedic modules 3 that correspond to body parts. Orthopaedic modules 3 are equipped with fixatives 4 for fixation on respective body parts and are actuated by the drives subsystem (not specified in Figure 1). The system also has the control hardware and software 5 for spatial position change of each orthopaedic module 3 executed on the basis of a computer with a controller (not specified in the drawings) with the possibility of controlled connection to each orthopaedic module 3 via the corresponding drive. The system also has feedback means based on the sensors (not specified in Figures 1) of human body physiological indicators 1. The controlled movable frame 2 is kinematically connected to the outer rigid fixed frame 6 made as a three-dimensional frame structure defining a space for changing the position of orthopaedic modules 3 and forming a two-layer exoskeleton together with the controlled movable frame 2. Electromechanical drives subsystem 7 is located on the outer rigid fixed frame 6. Each of the drives is connected to the corresponding orthopaedic module 3 by a flexible connection 8. Each orthopaedic module 3 and each sensor of the human body physiological indicator 1 are connected to the control hardware and software 5 for spatial position change of each orthopaedic module 3 via the outer fixed frame 6. The control hardware and software 5 for spatial position change of each orthopaedic module 3 are also equipped with a virtual reality imaging subsystem configured to co-operate with the electromechanical drives subsystem. The two-layer exoskeleton is equipped with the initial suspension position fixation device 9 with support in the hip area and/or in the upper body area. Each orthopaedic module 3 is designed with an option for mechanotherapy of individual joints.

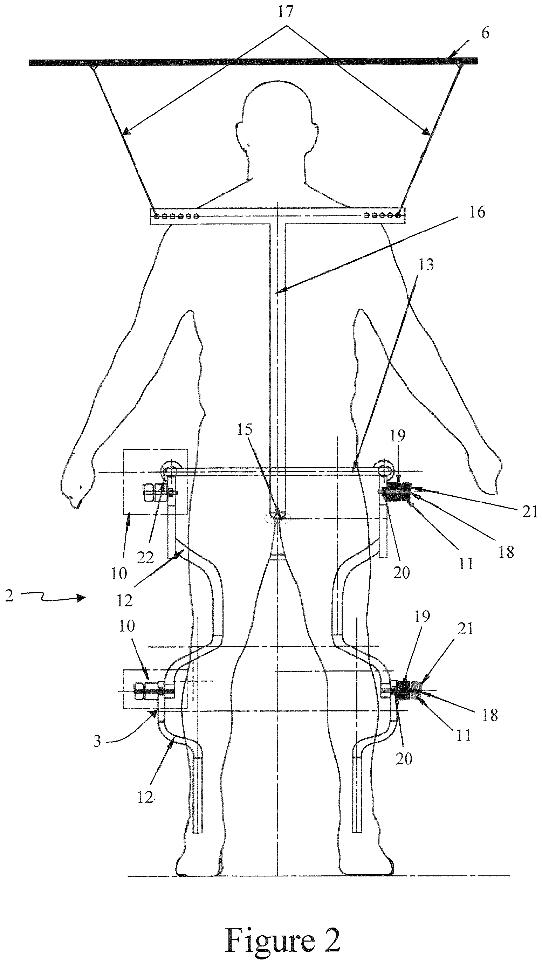

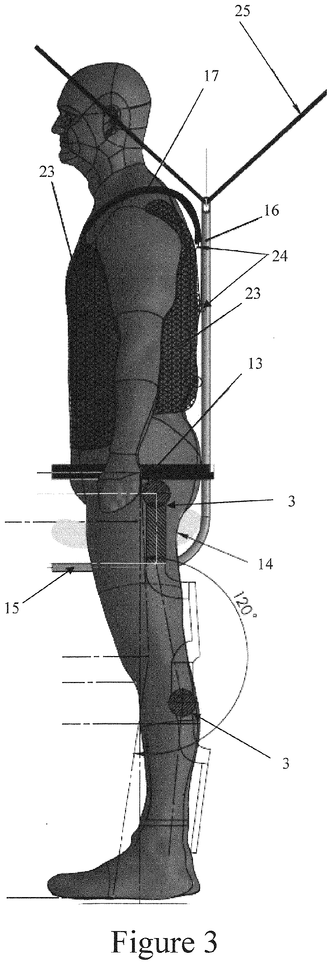

Figure 2 shows the rear view of the exoskeleton, controlled movable frame in one of the embodiments, and Figure 3 shows the side view. The orthopaedic modules 3 (femoral, knee, etc.) are kinematically interconnected by electromechanical parts 10 effected based on electric motors 11 connected with respective levers 12. Orthopaedic modules 3 (femoral, knee, etc.) can be designed to transmit vibrational and/or massaging influences to respective body parts. The corresponding means are not presented in the drawings in detail, but the experts in this field of invention can implement this function using suitable units and devices available.

The controlled movable frame can be equipped with additional fixtures and cushioning elements, as well as customization elements. The controlled movable frame 2, in the design presented as an example in Figures 2 and 3, is equipped with a pelvic fixative 13 and a cushioning pad 14 located on the support part 15 of the vertical support frame 16 with the suspension system 17. For example, knee, femoral module, etc. can be made cushioning. The support part 15 of the vertical support frame 16, as part of the initial suspension position fixation device, provides for the lower body support in the perinea region. At the same tine, the experts in this field of invention can generally make the initial suspension position fixation devices with a possible upper body support, for example, in the axillary region. Moreover, support (horizontal) sections of such devices can have cushioning pads. Similarly, the initial suspension position fixation devices can be made with a possible upper body support and lower body support in the perinea region with the possibility to adjust the distribution of the fixation percentage between the upper and lower parts, usually, in the automated or automatic mode through the hardware and software 5. Body fixation devices can also be equipped with cushioning components facilitating passive adduction of the body part towards a target point(s) of the rigid fixed frame 6.

Customization elements can include length adjustable levers 12, girth adjustable fixatives 4 of the orthopaedic modules 3, girth adjustable pelvic fixative 13, etc. All of these elements can be selected by the experts in the field of invention from the prior art or designed to be used in each particular exoskeleton, depending on its general design, conditions of use, etc.

To ensure performance of anatomically correct controlled motions, besides the electrical motors 11, orthopaedic modules 3 (knee, femoral, etc.) also have a drive shaft 18, a gear 19, a rate-of-turn sensor 20 of the drive shaft 18, a motor temperature sensor 21, etc. The levers 12 of the orthopaedic modules 3 are interconnected by power transmissions, for example by the joints 22.

Medical orthosis 23, which is put on the patient, is fixed in certain points 24 to the elements of controlled movable frame 2, for example to the vertical support frame 16 and the like. Generally, medical orthosis 23 is equipped with built-in sensors of the human body physiological indicators 1 (temperature sensor, pressure sensor, heart rate sensor, etc.), that are not shown in the drawings. These sensors are located in the medical orthosis 23 in such a way that the readings are transferred from them via the feedback channel through the controlled movable frame 2, flexible connections 8, the outer rigid fixed frame 6 to the hardware and software 5, where they are processed.

Item 25 shows the elastic suspension system elements connected (in the embodiment presented in Figures 2 and 3) with the outer rigid fixed frame 2.

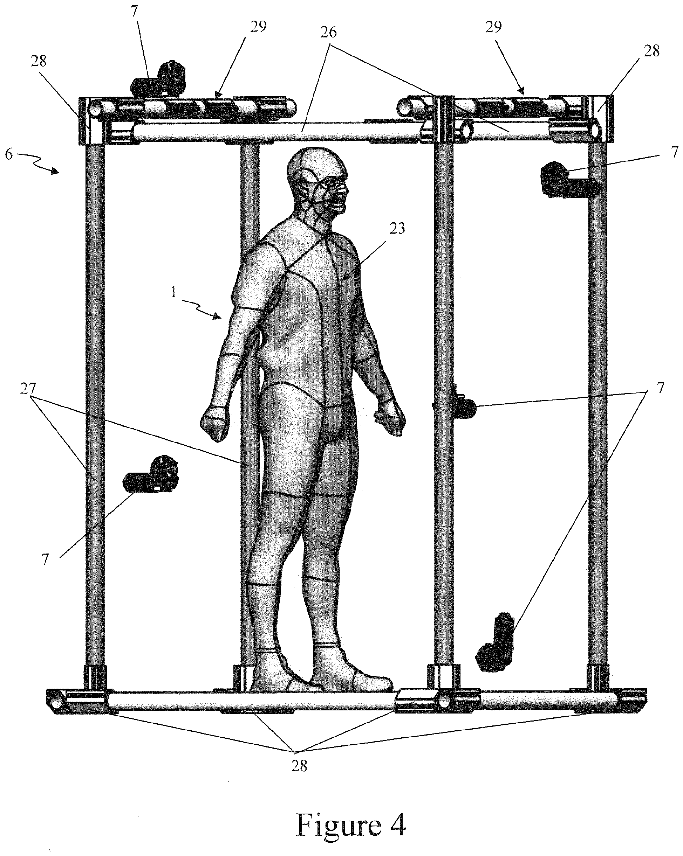

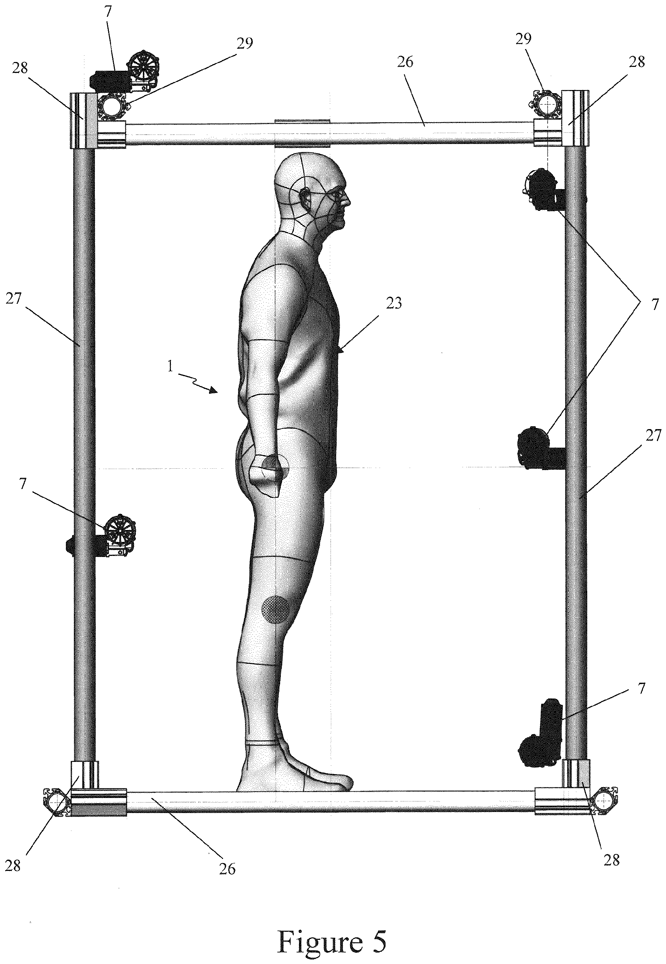

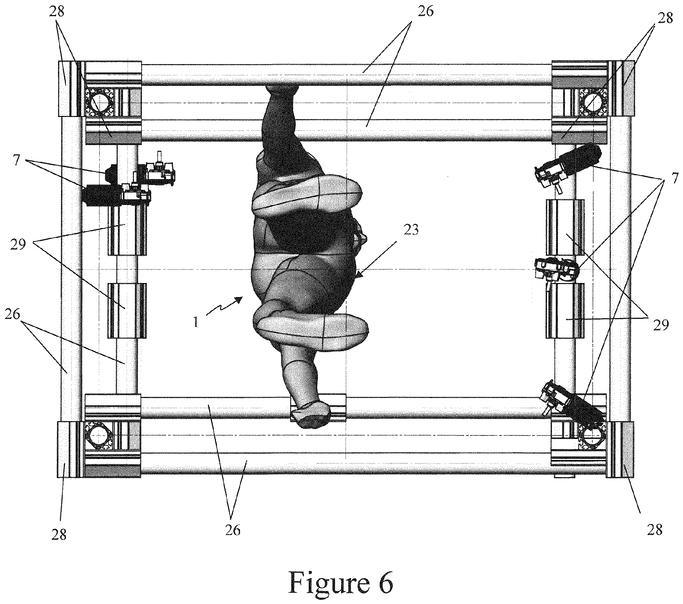

Figures 4-6 show different, projections of the outer external rigid fixed frame in one of possible embodiments.

The outer rigid stationary frame 6 is made as a three-dimensional frame structure of horizontal and vertical elements 26, 27, respectively, defining a space for changing the position of orthopaedic modules 3. In this case, the horizontal 26 and vertical 27 elements of the outer rigid fixed frame 6 are interconnected by angular 28 and upper 29 means for adjusting the geometric parameters of the space for changing the position of the orthopaedic modules 3. In the presented example, height H, width B, and depth L are considered as the parameters of the space for changing the position of the orthopaedic modules 3.Generally, the experts in this field of invention can choose any design of 28, 29 means, allowing each horizontal 26 and vertical 27 element to move with respect to the other interconnected elements 26, 27 in the direction of three coordinate axes for the angular 28 and two coordinate axes for the upper 29 means for adjusting the geometric parameters of the space for changing the position of the orthopaedic modules 3. In particular, the said 28, 29 means can have a telescopic design, as illustrated in Figures 4-6. Electromechanical drives 7 of the electromechanical drives subsystem are placed on the outer rigid fixed frame 6. In Figure 4, electromechanical drives part 7 is shown with no reference to the horizontal 26 and vertical 27 elements to illustrate the possibility of placing any number thereof, almost at any "point" of the fixed frame 6. Moreover, electromechanical drives 7 placed on the horizontal 26 and vertical 27 elements using special fasteners with articulated mechanisms provide for changing the angle between the respective element 26, 27 and directly the electromechanical drive 7 elements securing setting of the flexible connection 8. Flexible connections 8 and the controlled movable frame 2 are not shown in Figures 4-6.

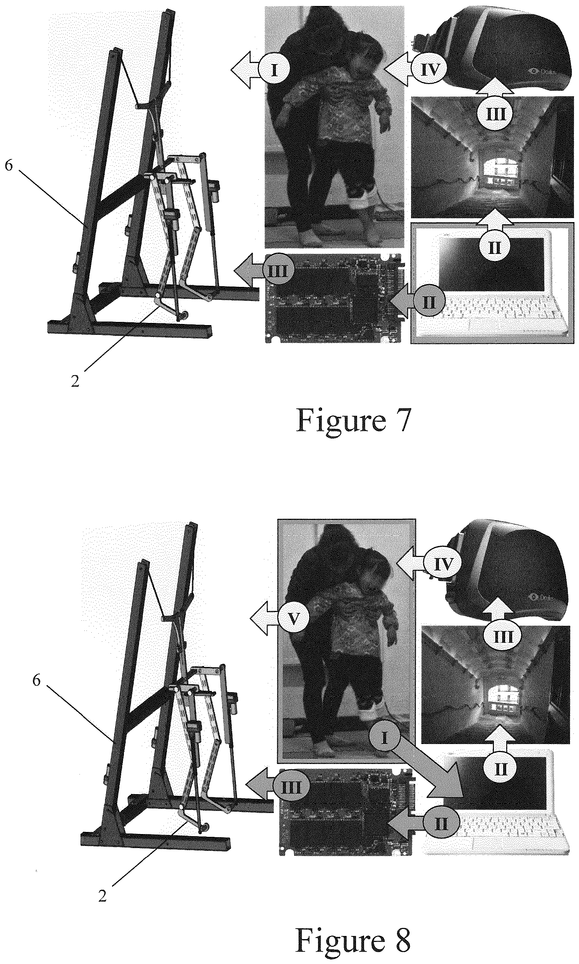

Figures 7 and 8 show the implementation schemes of two basic principles: "motor programming" and "motor control" respectively, underlying the claimed method of motor function recovery using adaptive kinesitherapy methods, using the claimed system for motor function recovery. Light arrows show the "sensory virtual event display channel," and the dark arrows show the "mechanical virtual event repetition channel."

Roman numbers in Figure 7 show the following stages of the "motor programming" principle as part of the claimed method: I - customization of the two-layer exoskeleton to the patient; II - virtual script run on the computer with transfer of its main events to the imaging system and controller; III - transfer of the virtual event to the "sensory virtual event display channel" and "mechanical virtual event repetition channel" (electromechanical drives system of the two-layer exoskeleton); IV - perception of the performed action.
Roman numbers in Figure 8 show the following stages of the "motor control" principle as part of the claimed method: I - mechanic arm(s) activation in response to a virtual event (for example: the need to evade an obstacle); II - transfer of this information to the controller and sensory imaging system; III - change in the spatial position of the two-layer exoskeleton and the corresponding visual information; IV - assessment of the ongoing sensory changes; V - perception of spatial movement.

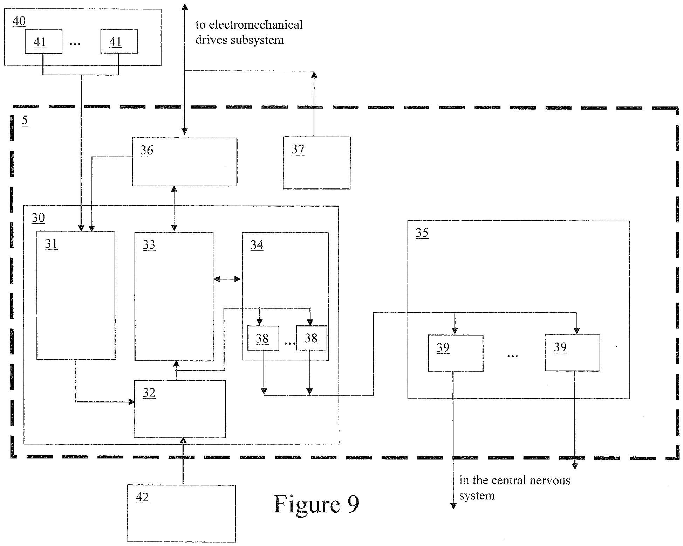

Figure 9 shows a simplified hardware and software 5 flowchart. Software and hardware include the following main units: personal computer 30, including data collection and analysis unit 31, influence program selection unit 32, spatial position change of each orthopaedic module 3 unit 33 and specific virtual reality environment creation unit 34; imaging subsystem 35; controller 36 and mechanic arm 37. In turn, the virtual reality creation unit 34 includes a number of "Assist" models 38. The imaging subsystem 35 includes at least one visualizer 39 connected to the virtual reality environment creation unit 34 and sends information to the patient's central nervous system in accordance with the imaged assist-model 38. Controller 36 and mechanic arm 37 are connected to the electromechanical drives subsystem. Data collection and analysis unit 31 is connected to the feedback means, including a number of the human body physiological indicators sensors 41, and via the controller 36 is connected to the electromechanical drives subsystem.

The control hardware and software 5 for spatial position change of each orthopaedic module 3 are designed to remotely record and/or correct the program for the coordinated functioning of the virtual reality imaging subsystem 35 and the electromechanical drives subsystem using relevant remote controls 42.

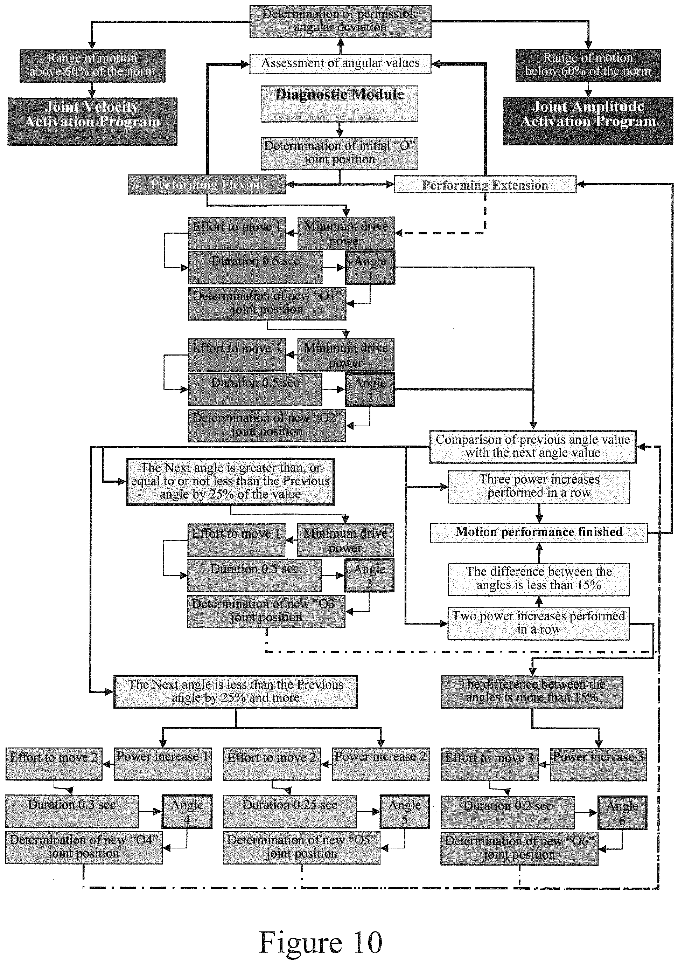

Figure 10 shows the algorithm for determining the viscoelasticity (the elastic barrier) of the locomotor system articulations by the control software and hardware 5 for spatial position change of each orthopaedic module 3 and feedback means (sensors 41). This algorithm is implemented in data collection and analysis unit 31.

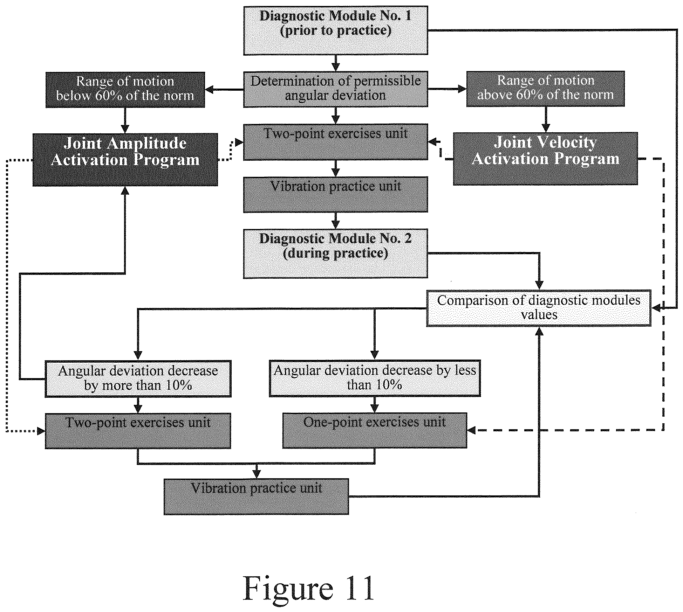

Figure 11 shows the algorithm for automatic selection of exercises performed on two legs (two-point exercises) and one leg (one-point exercises), which is also implemented in data collection and analysis unit 31.

The algorithms shown in Figures 10 and 11, are only examples of the functionality of the data collection and analysis unit 31 and the hardware and software 5 as a whole, and do not limit the capabilities of the latter.

The claimed method of human motor function recovery is implemented using the claimed system as follows.

The medical orthosis 23, equipped with built-in sensors of the human body physiological indicators, is put on person 1. The controlled movable frame 2 (exoskeleton) is put on the lower limb girdle and the upper shoulder girdle of person 1 and fixed on the human body, in particular in the area of the orthopaedic modules 3 using the corresponding fixatives 4, and also using the pelvic fixative 13. In this case, the medical orthosis 23 is fixed to the controlled movable frame elements 2, in particular to the vertical support frame 16 at the respective fixing point(s) 24 so that the lower body support 1 is formed in the perinea region by a horizontally oriented support part 15 of the vertical support frame 16 with the cushioning pad 14 placed thereon. The suspension system in the embodiments shown in the drawings is fixed to the horizontal elements 26 of the outer rigid fixed frame 6 with the elastic elements 25. The tension of the elastic elements 25, adjusted with the hardware and software 5, provides the gravity load/discharge values set for each particular patient and for each specific case.

The controlled movable frame 2 is connected (via the flexible connections 8) to the outer rigid fixed frame 6 made as a three-dimensional frame structure of the horizontal and vertical elements 26, 27 respectively, defining a space for changing the position of orthopaedic modules 3 and forming a two-layer exoskeleton together with the controlled movable frame. In this case, the controlled movable frame 2 is connected to the outer rigid fixed frame 6 by the flexible connections 8 via the corresponding electromechanical drives 7 located on the fixed frame 6. The horizontal 26 and vertical 27 elements of the outer rigid fixed frame 6 are interconnected by angular 28 and upper 29 means for adjusting the geometric parameters of the space for changing the position of the orthopaedic modules 3. The angular means 28 for adjusting the geometric parameters of the space for changing the position of the orthopaedic modules 3 allow each of the horizontal 26 and vertical 27 elements to move with respect to the other interconnected horizontal 26 and vertical 27 elements along all three coordinate axes. The upper means 29 for adjusting the geometric parameters of the space for changing the position of the orthopaedic modules 3 allow each of the horizontal 26 and vertical 27 elements to move with respect to the other interconnected horizontal 26 and vertical 27 elements along the two coordinate axes located in a horizontal plane. Due to this, in a wide range of values, the height H, the width B and the depth L of the space for changing the position of the orthopaedic modules 3, and also the spatial position of the electromechanical drives 7 with respect to the human body 1 (controlled movable frame 2/ medical orthosis 23) can be changed. In addition, by changing the position on the respective horizontal 26 or vertical 27 elements of the electromechanical drive 7, and also by changing (for example, using the appropriate articulated joint), including the one controlled in automatic/automated mode, the angular position relative to the said element 26 or 27, the unit for fixation of the flexible connection 8 of the electromechanical drive 7, it is possible to set the starting positions and the trajectories of the movement of the flexible communication 8 and the orthopaedic modules 3, as well as other controllable movable frame 2 elements in nearly unlimited range.

Electromechanical drives 7 are connected to the control unit (controller 36) and, if any, to the control module (mechanic arm) 37 from among the control hardware and software 5. Thus, the exoskeleton (controlled movable frame 2) is connected to the controller 36 and/or the mechanic arm 37, if any, from among the control hardware and software 5, directly activating (turn-on and start of various training motor programs and, accordingly, the change in the position electromechanical drives 7 and other actuators) the elements that change the position of orthopaedic modules 3, moving the body parts in the set mode (according to the requirements of the training program). In this case, the electromechanical drives 7, located on the fixed frame 6 (on the horizontal 26 and vertical 27 elements of the frame 6) form a gravity dosing system, which:
- facilitates the patient lifting,
- provides the patient's gravity load/discharge dosing by controlling the electromechanical drives 7 position relative to the space to change the position of the orthopaedic modules 3 and "tensing" the elastics elements 25 and flexible linkages 8,
- ensures a certain patient's spatial position and selected directions for the necessary movement,
- ensures environmental augmentation with displacement over 6 degrees of freedom (roll, pitch and yaw),
- creates a potential connection to a full-fledged virtual reality system.

The features of the "two-layer" design of the exoskeleton described above provide a highly effective system for customization to a patient, including his or her anthropometric data, as well as to a wide range of implemented techniques, different requirements, conditions and uses.

An additional advantage of the claimed system is the presence of feedback means (the human body physiological indicators sensors 41), as well as the rate-of-turn sensor 20 of the shaft from among the electromechanical drives 7, etc., which provide for the possibility to perform diagnostics in the automatic/automated mode, for example in accordance with the algorithm for determining the viscoelasticity of the locomotor system articulations presented in Figure 10, processing of the data obtained from the feedback means (sensor 41) and the controller 36 in the data collection and analysis unit 31 and selection of the influence program in the corresponding unit 32. Diagnostics can be continuously carried out during the practice (performance of the previously selected influence program) with automatic/automated correction of the influence program based on the results of processing data coming to the data collection and processing unit 31. An example of making an automatic/automated correction of the influence program during practice is shown in Figure 11 as the algorithm for automatic selection of exercises performed on two legs (two-point) and one leg (one-point). In this case, the diagnostics possibility, in particular the determination of the viscoelasticity of the locomotor system articulations, in automatic/automated mode ensures the preparation and implementation of an individual influence program for each patient, which prevents inefficient and unsafe load forcing, in particular, performance of exercises in the modes (the choice of the range of change in joint angles) which still can not be performed by this patient.

The hardware and software 5 also has a specific virtual reality rehabilitative environment creation unit 34 with virtual augmentators as basic "Assist" models 38, as well as the virtual augmentators integrators as basic assist-models 38 with electrical and electromechanical kinetic trainer parts, made as a spatial position change of each orthopaedic module 3 control unit 33, which transmits via the controller 36 the appropriate control actions to the electromechanical drives subsystem to each electromechanical drive 7 and, further, to the controllable movable frame 2. Presence of these units provides an opportunity for effective spatially oriented simulation of various, primarily complex human motions using the claimed method of motor function recovery, which is positioned by the authors as a Smart Dosing technique of step-by-step creation of "rigid" matrices of motion pattern in the brain: two-level intelligent methodical training load dosing, made automatically for each patient based on the previous practices results and the current evaluation of the joints viscoelasticity. This technique, i.e. the claimed method of human motor function recovery, is based on two functioning principles of the claimed system-a robotic kinetic trainer-that are used according to certain schemes, namely:
1. The "motor programming principle" shown in Figure 7.
2. The "motor control principle" shown in Figure 8.
The "motor programming principle" involves parallel activation of two quasi-technical channels:
1. The "sensory virtual event display channel," shown in Figure 7 in light arrows, is ensured by the interrelations between the specific virtual reality rehabilitative environment creation unit 34 with the virtual augmentators as basic "Assist" models 38, the imaging subsystem 35 and the patient/trainee.
2. The "mechanical virtual event repetition channel," shown in Figure 8 in dark arrows, is ensured by the interrelations between the specific virtual reality rehabilitative environment creation unit 34 with the virtual augmentators as basic "Assist" models 38, virtual augmentators integrators as basic assist-models 38 with the electrical and electromechanical kinetic trainer parts made as a spatial position change of each orthopaedic module 3 control unit 33, and the patient/trainee.

The term "quasi-technical channel" used herein includes both purely technical channels/means and information channels/means, as well as the channels formed by the visual and kinesthetic information perception channels and the patient's/trainee's nervous system pathways, ensuring the basic "Assist" models 38 are transmitted from the virtual reality rehabilitation environment creation unit 34 to the patient's/trainee's CNS.

In the course of the "motor programming," which, in fact, constitutes the first stage of the claimed method, the following actions (steps) are performed:
1. Two-layer exoskeleton customization to the patient (adjustment of the limb coverage by orthopaedic modules 3 by adjusting the fixatives 4; adjustment, if any, of the length of the levers 12; adjustment of the tension of the flexible connections 8; adjustment of the suspension system 17 to provide the necessary gravity load/discharge, etc.).
2. Virtual script consisting of the basic "Assist" models 38 run on the computer 30 with the transfer of its main events to the imaging subsystem 35 and the controller 36.
3. Parallel transfer of the virtual event to the "sensory virtual event display channel" and the "mechanical virtual event repetition channel" with the transfer of the appropriate control action via the controller 36 to the electromechanical drives subsystem 7 to the electromechanical drives 7 of the two-layer exoskeleton installed on the outer rigid fixed frame 6, and to the electromechanical parts 10 (electric motors 11) of the orthopaedic modules 3.
4. The patient's perception of the performed action by comparing the visual and mechanical images of the performed motion(s).

At the last step during the performance of basic motions, the time-matched information received by brain from the complex receptor device, integrating with the information from virtual reality environment coming via the visual channel, creates an illusion of the motion that is imaged in the virtual reality environment. In the process of such repetitions, the brain starts to create a "rigid matrix" of the given motion, as of its own. As a result of training, basic motion pattern are neurosensory programmed in the brain. These motion patterns can then be arbitrarily used in various combinations, for the patient creating own motor programs and are the basis for implementing the second principle: the "motor control" principle.

As in the "motor programming" principle, the "motor control" principle involves successive activation of two quasi-technical channels:
1. The "mechanical virtual event repetition channel," shown in Figure 8 in dark arrows, is ensured by the interrelations between the specific virtual reality rehabilitative environment creation unit 34 with the virtual augmentators as basic "Assist" models 38, virtual augmentators integrators as basic "Assist" models 38 with the electrical and electromechanical kinetic trainer parts made as a spatial position change of each orthopaedic module 3 control unit 33, and the patient/trainee.
2. The "sensory virtual event display channel," shown in Figure 8 in light arrows, is ensured by the interrelations between the specific virtual reality rehabilitative environment creation unit 34 with the virtual augmentators as basic "Assist" models 38, the imaging subsystem 35 and the patient/trainee.

In the course of the "motor programming," which, in fact, constitutes the second stage of the claimed method, the following actions (steps) are performed:
1. Mechanic arm(s) 37 activation in response to a virtual event (for example, the need to evade an obstacle).
2. Transfer of this information to the controller 36 and sensory imaging subsystem 35.
3. Change in the spatial position of the two-layer exoskeleton and the corresponding visual information.
4. Assessment of the ongoing sensory changes.
5. Perception of spatial movement.

A simplified flowchart of the hardware and software 5 as part of the claimed system for human motor function recovery, shown in Figure 9, contains only the main units and connections, which have a specific design to solve the tasks set to the claimed system and have the optimal embodiment of the claimed method. In addition to the specific units and connections, the software and hardware 5 as part of the claimed system also include the units and connections standard for control systems that ensure the overall system availability.

It should also be noted that the author carried out a detailed comparative analysis and assessment of the functionality of Lokomat and ReoAmbulator training devices, discussed above, and the system offered by him as a robotic trainer called TriNiTi. Comparison results by a number of essential features and functionality is given in the Table 1 below..

**Table 1. Comparison of kinetic trainers features and functionality**

| FUNCTION | System of the invention | Lokomat/ ReoAmbulator |
|---|---|---|
| Influence on one joint | + | - |
| Influence on several joints at the same time | + | + |
| Practice in horizontal position | + | - |
| Practice in vertical position | + | + |
| Practice under dosed gravity (support function dosing) | + | + |
| Variety of motor images (Assist models) | + | - |
| Neuro-biomechanical dosing of motor images intensity | + | - |
| Multilevel step-by-step dosing by physical factors | + | - |
| Virtual Trainer function | + | - |
| Remote Trainer function | + | - |
| Multiuser design | + | + |
| Customization | + | - |
| No unit specificity | + | - |
| Design-provided cushioning action | + | + |
| Pelvic and thoracic fixation | + | + |
| Motivational correction | + | + |
| Spatial orientation practice | + | + |
| Continuous Passive Motion therapy | + | - |
| Coordination practice | + | - |
| Vibration practice | + | - |
| Complex locomotion/Motion sensors | +/+ | -/+ |

Thus, the comparative table clearly shows the advantages of the claimed system and method of motor function recovery in comparison with the closest analogs by a big number of characteristics and confirm the possibility of active and passive kinesitherapy techniques performance using the claimed system, imitating the activation, control and assessment of the results achieved for various motions, complex ones in the first place.
The implementation of the claimed method of motor function recovery will be further illustrated with some non-limiting examples.

### Examples

### Example 1. Method of Motor Function Recovery in Parkinson's Disease

**Patient S. 57 years old** with a diagnosis of Parkinson's disease, akinetic-rigid form, stage 2.5 by Hoehn and Yahr, was examined on an outpatient basis. In a clam state, the patient had visual signs of a postural imbalance, manifested by postural changes. Also, the patient had postural instability of retropulsion when performing the jogging test. The patient has clinical signs of hypokinesia, manifested, first of all, by a decrease in the total scope of simple motions performed, as moderate motor deficits and walking disorders. The patient had a resting tremor-a small-amplitude tremor combined with a mild postural tremor. When assessing the muscle tone condition, the patient had a moderately pronounced extrapyramidal change with the left half involvement. There was sufficient direct and consensual papillary light reflex with a slight weakening of accommodation response to convergence. Mild asymmetry of tendon and periosteal reflexes with a positive Puusepp reflex. Prior to adaptive kinesitherapy sessions using the motor function recovery system as part of the implementation of the claimed method of motor function recovery, the quality parameters of the patient's spepping motion were impartially assessed using the claimed system (primarily hardware and software 5) embodied in Step functionally complicated version using a video capture system. At the same time, a sensor in the form of an active LED marker was placed in the common center of gravity projection area. When the diagnostic motion was performed, the spatial movement of the marker was video recorded, further, then the Psign (goal directed motion indicator) and the Pnoise (purposeless motion indicator), as well as the Motion Utility Coefficient were calculated in the data collection and analysis unit ... (video sequence processing program). The diagnostics had the following indicator values: Psign - 8,561.5; Pnoise - 560.6; Motion Utility Coefficient - 6.6.

These indicators reflect the effectiveness of a complex, standardized locomotion predominantly stabilized in the frontal and sagittal planes. In this case the reference values for healthy persons are: Psign - 7,840.1 [10,160.1/,5864.6]; Pnoise - 60.7 [102.9/45.4]; Motion Utility Coefficient - 0.9 [1.4/0.6].

Based on the indicators assessment results in accordance with the "selection" of the unit ... the influence program selection, 20 adaptive kinesitherapy sessions were prescribed for the patient and conducted according to the following scheme, consisting of two stages:

Stage 1. Consisted of 12 daily procedures, during which the patient was fixed in a controlled movable frame of a two-layer exoskeleton. Given the peculiarities of the disease associated with the left-sided lateralization of motor disorders and the diagnostics results, the influence program selection unit 32 selected (from the hardware and software 5) a stepping motion (as the initiating unit) with support on the right leg and placing the left leg forward (frontal lunge of the left leg), with its subsequent return to the original position to the supporting leg. In the current training program, this motion was a trained motion pattern, the image of which was restored in the central nervous system. At the same time, via the visualizer 39, a virtual reality environment was transmitted to the patient, where the patient moved forward with each activation of the movable frame 2 elements of the two-layer exoskeleton, by activating the electromechanical drives 7 located on the outer rigid fixed frame 6. The structure of this virtual motion corresponds to the motion forcibly performed in real space. During 12 sessions, the control program individually increased the joint kinematics amplitude and kinematic velocity, according to the developed algorithm.

Stage 2. Consisted of 8 daily procedures, during which there was a recovery of connections between the surrounding events in the virtual reality environment and the structure of the previously recovered stepping motion pattern. In this case, the patient was fixed in a controlled movable frame 2 of a two-layer exoskeleton. At the same time, a wireless mechanic arm 37, allowing controlling the virtual reality events, was put in the right hand, which was complete in terms of complex motion performance. A task with moving steps, the patient had to step over, was transmitted in the virtual reality environment via the visualizer 39. As the step approaches, the patient moves his right arm forward with the mechanic arm 39, while stepping takes place in the virtual reality, and the movable frame 2 of the exoskeleton activates the stepping motion with a lunge forward and return to its original position. If the patient fails to timely react to the virtual reality environment stimulus, the control program simulated the fall due to activation of the frame drives 7 of the exoskeleton.

The effectiveness of complex motions was assessed at the end of the course using the video motion analysis. The following results were obtained: Psign - 7,661.1; Pnoise - 210.5; Motion Utility Coefficient - 2.7.

Thus, adaptive kinesitherapy sessions performed in accordance with the claimed method using the claimed system (kinetic trainer), objectively influenced the qualitative characteristics of complex stepping motion and improved its efficiency, mainly in the sagittal plane. The obtained data indicate that a stable stepping pattern matrix is being created.

### Example 2. Method of Motor Function Recovery in the Demyelinating Disease of the Central Nervous System

**Patient V. 36 years old** with a diagnosis of "demyelinating disease of the central nervous system" was examined outside exacerbation on an outpatient basis. The patient had coordination disorders of limb ataxia, asynergic motions, dysdiadochokinesia and hypotonia with predominant left-side involvement. The patient did not have a spastic muscle tone increase, nor any motor impairment. At the same time there was a left-sided pyramidal insufficiency. The patient had pathological reflexes in the form of pathological foot signs. Also, stem structures lesions were identified: binocular nystagmus, mild internuclear ophthalmoplegia. Sensitive disorders were bilateral with an emphasis on the dissociated type. Prior to adaptive kinesitherapy sessions using the kinetic trainer, the quality parameters of the patient's stepping motion were impartially assessed in Step functionally complicated version using a video capture system. The diagnostics had the following indicator values: Psign - 8,332.9; Pnoise - 10,257.2; Motion Utility Coefficient - 123.1.

The patient had 28 adaptive kinesitherapy sessions according to the following scheme consisting of two stages, the structure of which was described above. Given the peculiarities of the disease and the preliminary diagnosis results, hip abduction motion pattern was selected as the initiating unit at the first stage, which consisted in the right leg abduction to the side (to the right) and returning it to the original position. This motion allows optimizing the support function on the left side. At the second stage, solution of the task associated with deviating away from flying objects was selected as a virtual reality environment. At the same time, the virtual reality mechanic arm 39 was fixed in the common center of gravity projection area.

The effectiveness of complex motions was assessed at the end of the course using the video motion analysis. The following results were obtained: Psign - 8,951.5; Pnoise - 90.3; Motion Utility Coefficient - 10.

Thus, adaptive kinesitherapy sessions performed in accordance with the claimed method using the claimed system (kinetic trainer), objectively influenced the qualitative characteristics of the motion associated with hip abduction and building the contralateral limb support function, and improved its efficiency, mainly in the frontal plane. The obtained data indicate that a stable motion pattern matrix is being created.

### Areas of Application

Potential areas of use of the claimed system and method of human motor function recovery include: rehabilitation; traumatology and orthopaedics; neurology (child neurology), neurosurgery: neurorehabilitation (post-operative recovery); geriatrics; sports medicine.

The technological, software and methodological innovations developed and applied in the claimed system and method of human motor function recovery allow using them as hardware and methodical tools of modern kinesitherapy for diseases accompanied by motor disorders such as: infantile cerebral palsy; the effects of brain injury, including the effects of strokes; neurodegenerative diseases (Parkinson's disease, demyelinating disorders); spinal injuries and lower limb injuries; severe movement disorders with spinal disc herniation; congenital neuromuscular and osteoarticular system diseases; osteoporosis (pathologic fractures); degenerative (age) changes in bones and joints (arthroses, osteoarthroses).

### Information Sources.

1. Advanced developments, R&D, inventions. Robotic Arms. Electronic resource "Made by Us." [Electronic resource] - June 10, 2015. - Access mode: http://www.sdelanounas.ru/blogs/35662/
2. Exoskeleton Used in Paraplegia. Electronic resource Eurodoctor.ru. [Electronic resource] - June 10, 2015. - Access mode: http://rehabilitation.eurodoctor.ru/exoskeletonparaplegia/
3. EXOATLET - Russian Exoskeleton. ExoAtlet project web-site. [Electronic resource] - May 21, 2015. - Access mode: http://www.exoatlet.ru/
4. EP Patent No. 1137378 B1, published on August 27, 2003.
5. Lokomat®Pro. Walking Skills Recovery. Rehabilitation and Mechanotherapy. Beka RUS Business Website. [Electronic resource] - May 21, 2015. Access mode: http://www.beka.ru/ru/katalog/vosstanovlenie-navykov-khodby/lokomat-pro/
6. Rehabilitation Equipment. Robotic Mechanotherapy with Biofeedback. ReoAmbulator. Electronic resource "Medicine and New Technologies." [Electronic resource] - October 1, 2015. - Access mode: http://www.mednt.ru/catalog/reabilitaciya-posle-insulta/robotizirovannayaterapiya/reoambulator/

## Claims

1. A system for human motor function recovery made as a robotic kinetic trainer with a whole-body exoskeleton, formed by a controlled movable frame, consisting of kinematically interconnected orthopaedic modules with a suspension system that correspond to body parts, designed with a fixation possibility on relevant body parts and actuated by the drives subsystem, the control hardware and software for spatial position change of each orthopaedic module executed on the basis of a computer with a controller with the possibility of controlled connection to each orthopaedic module via the corresponding drive, and the feedback means based on at least one sensor of at least one human body physiological indicator, **characterized in that** the controlled movable frame is kinematically connected to the outer rigid fixed frame made as a three-dimensional frame structure defining a space for changing the position of orthopaedic modules and forming a two-layer exoskeleton together with the controlled movable frame, there is also electromechanical drives subsystem on the outer fixed frame, where each of the drives is connected to the corresponding orthopaedic module via the flexible connection, each orthopaedic module and each sensor of the human body physiological indicator are connected to the control hardware and software for spatial position change of each orthopaedic module via the outer fixed frame, the control hardware and software for spatial position change of each orthopaedic module of the claimed system are also equipped with a virtual reality imaging subsystem configured to co-operate with the electromechanical drives subsystem, the two-layer exoskeleton is equipped with the initial suspension position fixation device with support in the hip area and/or in the upper body area.

2. The system according to Claim 1, **characterized in that** the virtual reality imaging subsystem has at least one visualizer, wherein the control hardware and software for spatial position change of each orthopaedic module are also equipped with a unit for creating at least one specific virtual reality environment connected to the visualizer to generate motor motion images.

3. The system according to Claim 1 and 2, **characterized in that** each orthopaedic module is designed with an option for mechanotherapy of individual joints and/or to transmit vibrational and/or massaging influences to respective body parts.

4. The system according to Claim 1 and 2, **characterized in that** the controlled movable frame is equipped with at least one additional element selected from the group consisting of at least the fixtures and cushioning elements as well as customization elements.

5. The system according to Claim 1 and 2, **characterized in that** the kinematic connection between the orthopaedic modules is ensured by electromechanical parts.

6. The system according to Claim 1 and 2, **characterized in that** the initial suspension position fixation device is designed with a possible lower body support in the perinea region.

7. The system according to Claim 1 and 2, **characterized in that** the initial suspension position fixation device is designed with a possible upper body support.

8. The system claimed in Clauses 1 and 2, **characterized in that** the initial suspension position fixation device is designed separately with a possible upper body support and lower body support in the perinea region with the possibility to adjust the distribution of the fixation percentage between the upper and lower parts.

9. The system according to Claim 1 and 2, **characterized in that** body fixation devices can also be equipped with cushioning components facilitating passive adduction of the body part towards at least one point of the rigid fixed frame.

10. The system according to Claim 1 and 2, **characterized in that** the control hardware and software for spatial position change of each orthopaedic module are designed to remotely record and/or correct the program for the coordinated functioning of the virtual reality imaging subsystem and the electromechanical drives subsystem.

11. The system according to Claim 1 and 2, **characterized in that** the control hardware and software for spatial position change of each orthopaedic module also have a control module in the form of a mechanic arm, designed with a possibility of manual control by the trainee by a spatial position change of each orthopaedic module.

12. A method for human motor function recovery, including preparation of an individual training motor recovery program and creation of motion pattern in the central nervous system according to the prepared program by forced relevant change of the spatial position of body parts using the human motor function recovery system including the exoskeleton connected to computer-based controls **is characterized in that** any system claimed in Clauses 1-11 is used as a system for human motor function recovery, while recovery is carried out in two stages, when at the first stage, stable motion pattern matrices are created or restored in the central nervous system; at the same time, a motor image of at least one virtual motion is generated (in accordance with the individual training motor program), which is visualized and transmitted through the visual channel to the trainee's central nervous system, while a control action corresponding to this motion is transmitted to the controlled movable frame of the exoskeleton that forces at least one corresponding body part to move over and over again, and at the second stage, the connections between the surrounding events and the recovered motion pattern matrices are restored as responses to these events; at the same time, a motor image of at least one virtual event requiring a motor response is generated, which is visualized and transmitted through the visual channel to the trainee's central nervous system, followed by a control action corresponding to this motor response transmitted to the controlled movable frame of the exoskeleton that forces at least one corresponding body part to move over and over again, and by doing so, the trainee's condition, in particular the condition of the locomotor system, is monitored using feedback.

13. The method according to Claim 12, **characterized in that** the training motor program is prepared taking into account the load dosage and complicating the training motions starting with simple ones, performed in lying or standing position.

14. The method according to Claim 12 and 13, **characterized in that** the training motor program is prepared in a way that motor images are created by complicating them from static and statnamic to dynamic ones.

15. The method according to Claim 12 and 13, **characterized in that** the training load is dosed based on previous and current practice results, as well as on dynamic (determined during the practice) assessment of the trainee's locomotor system active structures viscoelasticity in automatic or manual mode.

16. The method according to Claim 12 and 13, **characterized in that** the training motor program is prepared taking into account motion space limitations due to the virtual component, which directs the virtual motion towards the motion space limitation, thus forming an idea in the patient's central nervous system of the potential readiness of the locomotor system to use this part of the motion space.

17. The method claimed in Clauses 12 and 13, **characterized in that** the training motor program is prepared taking into account motion space limitations due to the controlled movable frame of the exoskeleton, using which the practiced motion is oriented towards the motion space limitation, thus forming an idea in the patient's central nervous system of the potential ability of the locomotor system to use this part of the motion space.

18. The method according to Claim 12 and 13, **characterized in that** the training motor program is prepared taking into account motion space limitations due to the virtual component and the controlled movable frame of the exoskeleton, which together strengthen the motion matrix oriented towards motion space limitation.

19. The method according to Claim 12 and 13, **characterized in that** the active practice is provided with overcoming the resistance of the cushioning components facilitating passive adduction of a body part due to an arbitrary movement of the body part in at least one direction of the motion pattern matrix.
